# EUROPEAN PATENT APPLICATION

(11) **EP 0 832 666 A2**
(43) Date of publication of application: **01.04.1998**
(21) Application number: 97307160.8
(22) Date of filing: 15.09.1997
(51) Int. Cl.: A61M 39/04, A61M 39/20

(54) **Detachable blood seal**

(30) Priority: 30.09.1996 US 724558
(71) Applicant: Becton, Dickinson and Company, Franklin Lakes, New Jersey 07417-1880 (US)
(72) Inventor: Erskine, Timothy J., Sandy, Utah 84094 (US); Howell, Glade H., Sandy, Utah (US)
(74) Representative: Ruffles, Graham Keith

(57) **Abstract**

A removable blood seal is provided for preventing the leakage of blood during the placement and use of vascular catheters and similar devices. The structure of the blood seal includes a housing having a proximal end and a distal end. The distal end is configured such that a catheter can be removably attached to the distal end. The proximal end is configured such that a needle hub can be fit to the proximal end. The housing includes an internal channel disposed through the housing and configured such that a needle can pass through the channel. An elastomeric seal is placed securely within the channel such that it substantially blocks fluid flow through the channel. The seal is preferably configured of an elastomeric material such that if a needle penetrates said seal and is then later removed, said seal substantially closes the internal channel to fluid flow.

## Description

### BACKGROUND

### 1. The Field of the Invention

The present invention is related to a detachable blood seal for use in connection with various medical devices. More specifically, the present invention relates to a detachable blood seal which is particularly adaptable for use in sealing a vascular catheter immediately following placement of the catheter within a vein or artery of a patient.

### 2. Technical Background

Vascular catheters for infusion of fluids into patients are among the most commonly used medical devices. The insertion of a vascular catheter allows repeated or continuous access to the circulatory system of a patient. Vascular catheters are generally inserted into the extremities of a patient and fluids and medications are introduced to the patient through such catheters.

Catheters of this type are generally inserted into a vein or artery by means of an introducer needle. In one common configuration, the catheter is initially placed over the needle. The needle, with the catheter located over the needle, is inserted into the patient until the desired vein or artery is located. Once the needle and catheter are properly located in the vein or artery, the needle is withdrawn from the catheter and the vein or artery and discarded. The catheter remains in the vein or artery to provide access to the circulatory system of the patient without repeated needle punctures.

When the catheter insertion and placement steps have been concluded, one end of a tube (or "administration set") is generally attached to the proximal end of the catheter. The opposite end of the tube is attached to a source of fluid and medication. The source of fluid is typically a bottle or bag containing the fluid required for treatment of the patient. Once attachment of the catheter to the fluid source is completed, fluids are allowed to flow through the tubing, into the catheter, and ultimately into the patient. In most situations, fluids flow through the tubing set and into the patient by means gravity feed or using a standard infusion pump.

It will be appreciated that it is important to minimize the leakage of blood to the outside environment during each of the steps described above. Blood leakage can expose medical personnel and others to blood-borne diseases, such as AIDS and hepatitis. Blood leakage can contaminate equipment and supplies in the treatment area. Blood leakage may also cause unnecessary alarm on the part of the patient and other observers. Thus, it is important to prevent or minimize blood leakage in order to maintain safety, aesthetics, and to retain equipment and supplies in good working order.

Several of the steps necessary for the placement and use of catheters of the type described above involve the potential for blood leakage. One such step is the removal of the introducer needle once the catheter is in place. This step generally results in a short period of time during which blood may flow out of the catheter to the surrounding environment. Another step which may potentially result in blood leakage is the attachment of the tubing set to the catheter. These manipulations are complicated because the medical professional may be faced with performing multiple tasks and dealing with multiple pieces of equipment virtually simultaneously. Stated simply, the medical professional may not have enough hands to adequately control blood leakage while performing other necessary functions.

Attempts have been made to deal with the problem of blood leakage during the placement of catheters. However, those proposed solutions generally involve devices which are complex and expensive. For example, one such device employs a relatively complex valve mechanism connected to the proximal end of the catheter which opens when a needle is inserted and then closes when the needle is removed. The valve then opens again when the tubing set is attached to the catheter. This device requires a mechanism for repeated opening and closing of the valve, as well as other collateral structures which facilitate operation of the device. These relatively complex structures complicate the device and add to its cost. In addition, the valve device is designed to remain in place after the tubing set is attached. This increases the potential for irritation and discomfort to the patient.

Accordingly, it would be an advancement in the art to provide a device which would control the flow of blood during the steps surrounding placement and use of a catheter. It would also be an advancement in the art to provide such a device which is inexpensive and simple to operate. It would also be an advancement in the art to provide such a device which could be used without requiring significant modification of conventional catheters, needles, tubing sets and the like. Finally, it would be a significant advancement in the art to provide such a device which provided the medical professional with more control in performing the tasks surrounding placement and use of a catheter.

Such apparatus are disclosed and claimed herein.

### BRIEF SUMMARY AND OBJECTS OF THE INVENTION

The present invention is a removable blood seal for preventing the leakage of blood during the placement and use of vascular catheters and similar devices. In one presently preferred embodiment the blood seal has a housing which is substantially cylindrical in configuration. A catheter and an introducer needle are attached to the housing during the catheter placement steps. By using the present invention, medical professionals are provided with increased flexibility in the placement and use of vascular catheters and are able to minimize blood leakage.

The housing has an exterior with a distal end formed such that a catheter adapter can be removably attached to the distal end. Various conventional methods of attachment may be used. For example, the catheter adapter may be attached to the housing by means of friction engagement, a luer lock mechanism, or any other suitable means. The housing also has a proximal end configured such that a needle hub to which the introducer needle is connected can be removably attached. Various conventional means of attachment may be used here as well, including friction engagement and luer lock mechanism. In one presently preferred embodiment, the means of attaching the needle hub to the housing comprises a slot disposed through the housing, which in turn corresponds to a latch on the needle hub. The latch locks within the slot during placement of the catheter and is held in place by the needle. Once placement is complete and the needle is removed, the latch can disengage from the slot, allowing the needle hub and thus the introducer needle to be removed.

The blood seal also has an internal channel disposed within the housing which is configured such that the introducer needle can pass through the channel. An elastomeric seal is secured within and substantially blocks fluid flow through the channel. The seal is preferably formed from an elastomeric material such as rubbers and synthetic elastomers including latex, polyisoprene, silicone, and polyurethanes. The seal is configured such that when the introducer needle penetrates the seal and is then later removed, the seal substantially closes the internal channel to fluid flow.

The blood seal may have other optional features. For example, in certain contexts it is helpful to have a push tab disposed on the exterior of the housing. This allows the user to more easily and accurately maneuver the entire catheter mechanism during placement of the catheter. This may be important during the difficult and delicate steps of catheter placement. Thus, certain embodiments of the device optionally include such a push tab.

Thus, the present invention is useful in controlling blood leakage during the placement and use of vascular catheters. The present invention provides increased flexibility in the timing of the various steps involved in that it provides a barrier to blood flow. Thus, the problems related to blood leakage to the ambient environment are substantially reduced.

### BRIEF DESCRIPTION OF THE DRAWINGS

In order to more fully understand the manner in which the above-recited advantages and objects of the invention are obtained, a more particular description of the invention briefly described above will be rendered by reference to specific embodiments thereof which are illustrated in the appended drawings. Please take note that the embodiments illustrated in the drawings are merely illustrative.

Figure 1 is a perspective view of one embodiment of the present invention illustrating an introducer needle being inserted through the blood seal.

Figure 2 is a partially cutaway perspective view of the embodiment of the device illustrated in Figure 1 showing the catheter and introducer needle assembly attached to the blood seal.

Figure 3 is a cross sectional view of the assembly illustrated in Figure 2 taken along line 3-3 showing the manner in which the needle penetrates the seal.

Figure 4 is a perspective view of a further embodiment of the device in which the blood seal is pierced by an introducer needle. The embodiment illustrated in Figure 4 also includes a push tab and latch on the body of the blood seal.

Figure 5 is a partially cutaway perspective view of the embodiment of the device illustrated in Figure 4 where the introducer needle assembly is shown locked in place within the housing of the blood seal and the catheter is attached to the blood seal as well.

Figure 6 is a cross sectional view of the device illustrated in Figure 5 taken along line 6-6 of Figure 5.

Figure 7 is a further cross sectional view of the device showing the introducer needle retracted, the seal in place, and the latch in the retracted position.

Figure 8 is a cross sectional view of a further embodiment of the present invention including a rigid push off tab.

Figure 9 is a cross sectional view of a further embodiment of the device comprising a single elastomeric member and having a pull tab.

### DETAILED DESCRIPTION OF THE PREFERRED EMBODIMENTS

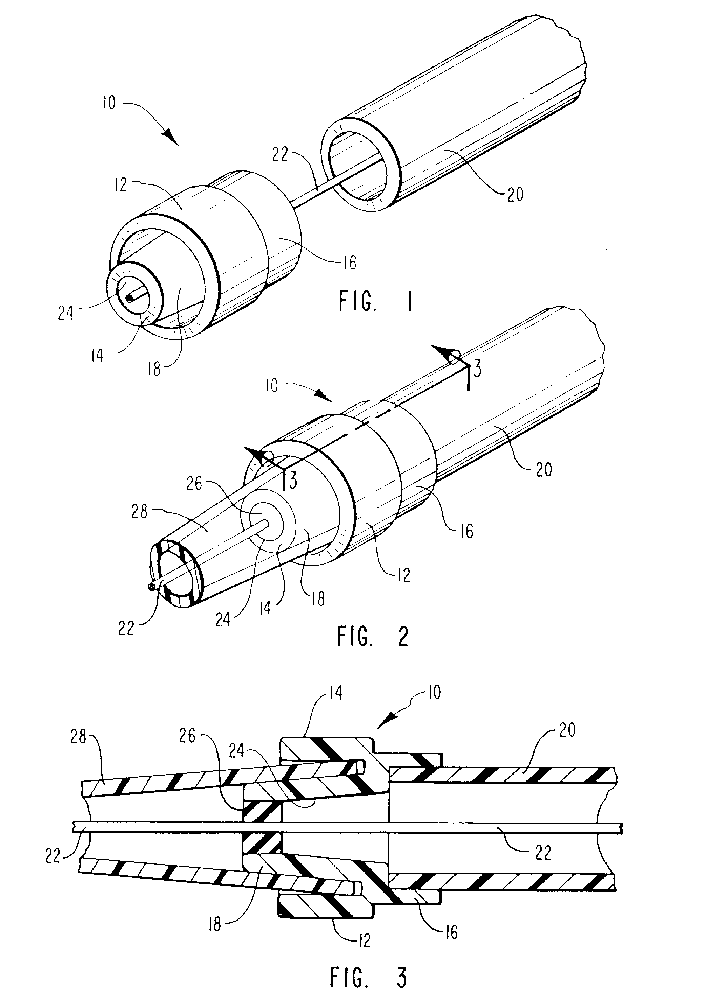

The present invention can be best understood by reference to the drawings where like parts are designated with like numerals throughout. One embodiment of the detachable blood seal of the present invention is generally designated 10 in Figure 1. As mentioned above, the blood seal 10 allows for the placement of a catheter in a patient and subsequent removal of the introducer needle while maintaining a blood seal at the proximal end of the catheter. This allows the medical professional a time interval to prepare for the attachment of a tubing set on the catheter without the need to be concerned about blood leakage through the catheter.

As illustrated in Figure 1, the blood seal 10 includes a generally cylindrical body 12. The body 12 has a distal end 14 and a proximal end 16. The distal end 14 is specifically configured such that a catheter adapter can be removably attached to the distal end 14. When using the embodiment of the device illustrated in Figure 1, the catheter is attached in friction engagement to the frustoconical attachment portion 18. However, other means of engagement may also be used, including threaded engagement and luer locking mechanisms.

The body 12 of the blood seal 10 also includes a proximal end 16. Proximal end 16 is configured such that a needle hub 20 to which the introducer needle 22 is attached can be removably attached to the body 12. As with the attachment portion 18, needle hub 20 will be attached by friction engagement in the embodiment illustrated in Figures 1 through 3. However, it is aiso possible to use other conventional or nonconventional attachment means as will be discussed in further detail below.

It will be appreciated that when the needle hub 20 is secured to the body 12, the associated introducer needle or cannula 22 passes through a channel 24 disposed within the body 12 of the blood seal 10. As will be discussed in further detail below, the needle 22 passes through an elastomeric seal 26 which is securely disposed within the channel 24. Thus, the needle 22 is prepared for use in introducing a catheter into the vascular system of a patient.

Figure 2 illustrates the blood seal 10 attached to a catheter adapter 28 and needle hub 20. As was briefly discussed above, the catheter adapter 28 is attached by friction engagement to the distal end 14 of the body 12. Specifically, the catheter adapter 28 is slid into place over the frustoconical attachment portion 18 until it is adequately secured to the body 12 of the blood seal 10.

At the proximal end 16 of the body 12 a needle hub 20 is attached by friction engagement within the proximal end 16. When the needle hub 20 has been connected to the body 12, the needle 22 protrudes through the seal 26, as illustrated. The seal 26 is preferably an elastomeric seal securely disposed within the interior channel 24 of the body 12. The seal 26 fits snugly around the needle 22 when the needle is in the position illustrated in Figure 2. When the needle 22 is withdrawn from the seal 26, the seal 26 closes about the needle hole, thereby preventing leakage through the seal 26. Thus, the needle hub 20 can be detached without risking blood leakage.

The method of use of the blood seal 10 can be more fully appreciated with reference to Figure 3. Figure 3 is a cross sectional view of the blood seal 10 taken along line 3-3 of Figure 2. As with Figures 1 and 2, the body 12 of the blood seal 10 is illustrated. The distal end 14 and the proximal end 16 are also illustrated. A catheter adapter 28 is shown attached to the body 12 by friction engagement by means of the frustoconical attachment portion 18 of the distal end 14. As can be fully appreciated with reference to Figure 3, the catheter adapter 28 easily slides into place over the attachment 18. Likewise, when it is necessary to remove the blood seal 10 from the catheter adapter 28, it is a simple matter to slide the catheter adapter 28 off of the attachment 18.

As illustrated in Figure 3, a needle hub 20 is shown attached to the proximal end 16. The needle hub 20 and the proximal end 16 are attached by friction engagement. When in this position, the needle 22 penetrates the elastomeric seal 26. When the catheter adapter 28 and the needle hub 20 are secured to the blood seal 10 as illustrated in Figure 3, the needle 22 and catheter are ready for insertion into a patient.

In operation, the needle 22 is used to locate the desired position for insertion of the catheter. In most cases, this will be within a vein in the patient. Once the vein is located by the needle 22, the catheter 28 is slid over the needle 22 into place within the vein as the needle 22 is simultaneously retracted. The needle 22 is retracted by disengaging the needle hub 20 from the proximal end 16 of the body 12. The needle hub 20 is then moved away from the body 12. This in turn causes the needle 22 to retract through the seal 26. Ultimately the needle 22 is completely removed from the blood seal 10. Because of the elastomeric nature of the seal 26, the needle hole immediately closes forming a fluid-tight seal. Thus, blood leakage at this point in the procedure is eliminated.

The blood seal prevents blood flow through the catheter during further preparatory steps which may need to be taken by the medical professional. Once the needle hub 20 and needle 22 are appropriately disposed of, it is possible for the medical professional to take the necessary steps to attach a tubing set to the catheter adapter 28. This may be accomplished by placing pressure on the catheter downstream from the blood seal 10 to restrict the flow of blood out of the catheter. The blood seal 10 is then detached from the catheter adapter 28 and the tubing set immediately replaces the blood seal 10. In this manner blood leakage is effectively eliminated.

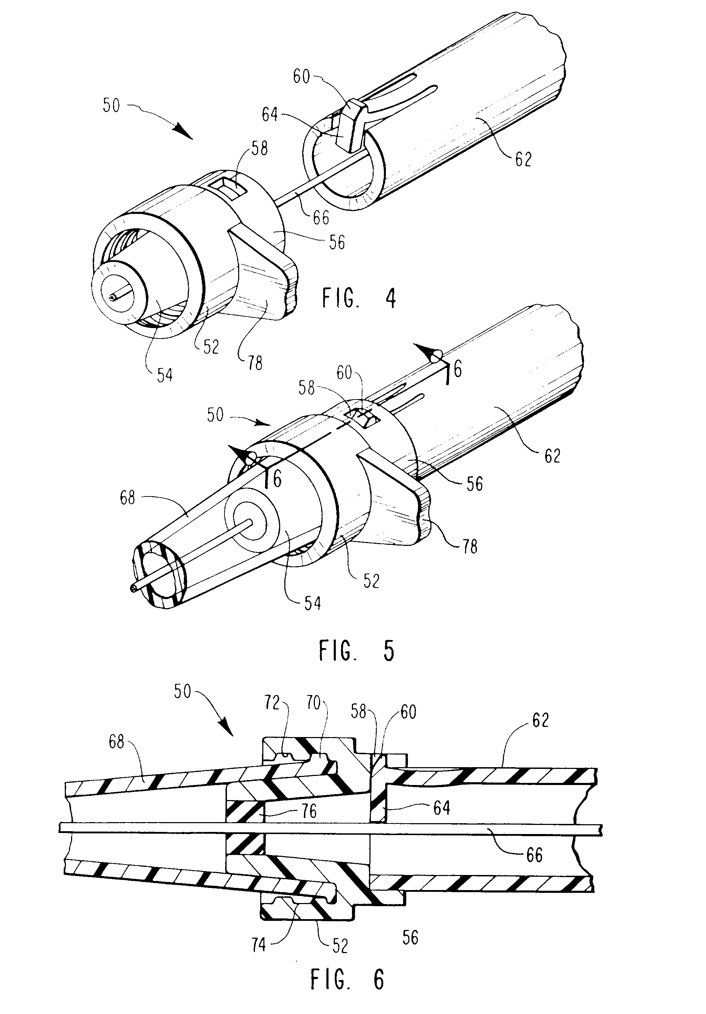

Turning now to Figure 4, an alternative embodiment of the device of the present invention is illustrated and generally designated 50. As with the previously described embodiment, blood seal 50 is comprised of a generally cylindrical body 52. The body 52 has a distal end 54 and a proximal end 56. Both the distal end 54 and proximal end 56 differ from those illustrated in Figure 1 through 3. Significantly, a slot 58 is disposed through the proximal end 56 of the body 52.

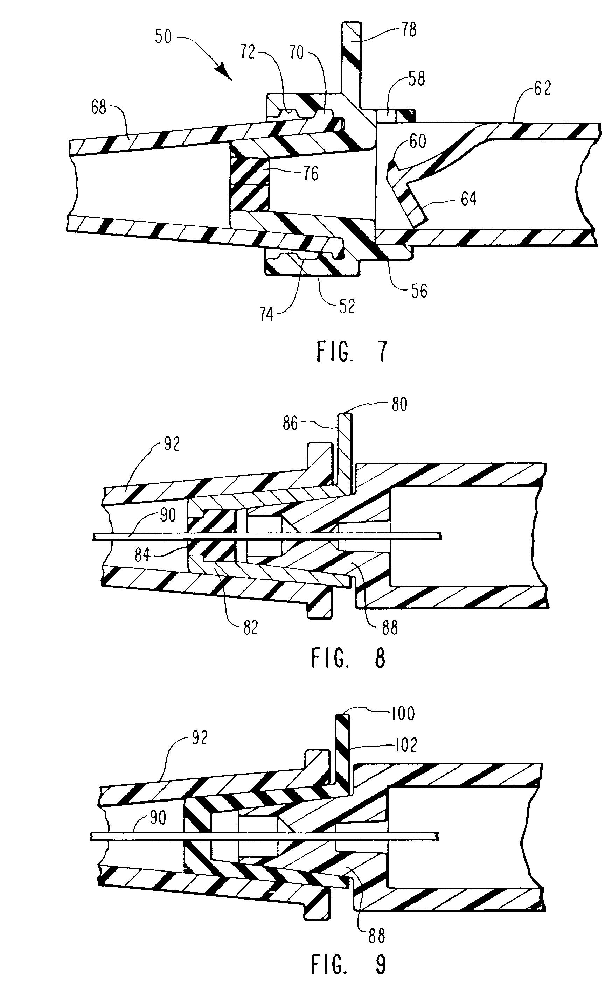

The slot 58 is configured such that it receives in locking engagement a portion of latch 60 which forms a portion of a needle hub 62. The manner of the engagement between the slot 58 and the latch 60 is best illustrated with reference to Figure 6. It can be appreciated that the latch 60 is urged into place within the slot 58 because an extension 64 of the latch 60 is held in place by the needle 66. As illustrated in Figure 7, when the needle 66 is removed, the latch 60 and extension 64 pivots downwardly. This allows the needle hub to be easily disengaged from the blood seal 50.

Referring to Figures 4 through 6, the manner of attachment of a catheter 68 adapter to the blood seal 50 can also be appreciated. In this embodiment, the flange 70 of the catheter adapter 68 is threaded into threads 72 in the distal end 54 of the body 52. This threaded engagement can be achieved by the use of a luer lock mechanism, or by other conventional threads. Thus, when it is desired to remove the body 52 from the catheter adapter 68, it is a simple matter to twist the body 52 until it disengages.

Also illustrated in Figures 4 through 6 is the needle 66. As in the previously described embodiment, the needle 66 penetrates and extends through an elastomeric seal 76. As mentioned above, the seal may be constructed of any one of a number of desirable materials. Examples of usable materials include rubbers and synthetic elastomers such as latex, polyisoprene, silicone, and polyurethanes. Essentially, any elastomeric material which forms a fluid tight seal when the needle 66 is removed is usable within the scope of the present invention.

Also illustrated in Figures 4 through 7 is tab 78. Tab 78 is positioned on the blood seal 50 such that it is usable to position the needle 66 and catheter. Tab 78 allows the user to more easily manipulate the device during the delicate steps of placement of the needle and catheter in a vein or artery.

Figure 5 illustrates the blood seal 50 assembled for use, with a catheter adapter 68 and a needle hub 62 attached. As discussed above, the catheter adapter 68 is attached by threaded engagement to the distal end 54 of the body 52. The needle hub 62 is attached by means of the slot 58 and latch 60 mechanism described above. Thus, the blood seal 50 as illustrated in Figure 5 is ready for use in introducing the catheter into a patient.

The needle 66 is inserted into the patient allowing the catheter to be positioned in a vein or artery. Once this is accomplished, the needle 22 may be removed as illustrated in Figure 7. The latch and extension mechanism are removed from the slot 58 because they are biased downwardly allowing the needle hub 20 to be removed from the body 52. Once this series of steps is completed, the catheter is in position and sealed by the blood seal 50. The medical professional can then remove the seal and attach a tubing set or the like at a convenient time.

Figure 8 illustrates a further embodiment of the present invention which is generally designated 80. The blood seal 80 is generally frustoconical in configuration. Blood seal 80 can be constructed of a wide range of acceptable materials well known to those of skill in the art. Blood seal 80 includes a body 82 and a seal 84. Seal 84 is similar in construction and materials to seals 26 and 76 described above. As illustrated in Figure 8, blood seal 80 includes a rigid push off tab 86 which allows the use to easily maneuver the overall device.

Also illustrated in Figure 8 are a conventional needle hub 88, needle 90, and catheter 92. The blood seal 86 is configured such that it can be attached to the catheter by friction engagement in the manner illustrated. Likewise, the needle hub 88 fits within the frustoconical form of the blood seal 86. When the device is assembled with an associated needle 90, hub 88, and catheter 92 the needle penetrates and extends through the seal 84. When placement of the catheter is complete, the needle hub is disengaged from the blood seal 80 and the needle slides through the seal 84. Seal 84 then prevents further fluid flow through the catheter 92 until the blood seal 80 is removed.

Figure 9 illustrates a further embodiment of the present invention generally designated 100. In this embodiment the device is a one piece elastomeric seal. In this configuration it is not necessary to provide a separate seal because the overall device is constructed of an elastomeric material. This embodiment of the device also includes a pull tab 102 for use in maneuvering the device. The operation of this embodiment of the invention is similar in nature to that of the embodiment illustrated in Figure 8.

In summary, the present invention provides a device which controls the flow of blood during the steps involved in the insertion of a catheter. The present invention provides a device which is inexpensive and simple to operate. The blood seal of the present invention can also be used without significant modification of conventional catheters, needles, tubing sets and the like.

The invention may be embodied in other specific forms without departing from its spirit or essential characteristics. The described embodiments are to be considered in all respects only as illustrative and not restrictive. The scope of the invention is, therefore, indicated by the appended claims rather than by the foregoing description. All changes which come within the meaning and range of equivalency of the claims are to be embraced within their scope.

## Claims

1. A removable blood seal comprising:
a housing;
an internal channel disposed through the housing and configured such that a needle can pass through said channel; and
a seal securely disposed in and substantially blocking said channel, said seal configured such that if a needle penetrates said seal and is then later removed, said seal substantially closes the internal channel to fluid flow.

2. A removable blood seal as defined in claim 1 wherein said housing is configured such that it can be securely placed between a catheter and a needle hub during placement of the catheter in a patient.

3. A removable blood seal as defined in claim 2 wherein said catheter is removably attachable to said blood seal by friction engagement.

4. A removable blood seal as defined in claim 2 wherein said catheter is removably attachable to said blood seal by means of a luer lock mechanism.

5. A removable blood seal as defined in claim 1 further comprising means for locking the housing to a needle hub.

6. A removable blood seal as defined in claim 5 wherein said means for locking the housing to a needle hub comprises friction engagement.

7. A removable blood seal as defined in claim 5 wherein said means for locking the housing to a needle hub comprises a luer lock mechanism.

8. A removable blood seal as defined in claim 5 wherein said means for locking the housing to a needle hub comprises a slot disposed in the housing which corresponds to a latch on said needle hub.

9. A removable blood seal as defined in claim 1 further comprises a push tab disposed on the exterior of said housing.

10. A removable blood seal as defined in claim 1 wherein sad seal is constructed of silicone rubber.
